# EUROPEAN PATENT APPLICATION

(11) **EP 2 045 602 A1**
(43) Date of publication of application: **08.04.2009**
(21) Application number: 08165799.1
(22) Date of filing: 03.10.2008
(51) Int. Cl.: G01N 33/50, G01N 33/555

(54) **Potency assay**

(30) Priority: 04.10.2007 DK 200701428
(71) Applicant: Symphogen A/S, 2800 Lyngby (DK)
(72) Inventor: Hansen, Anne Marie Valentin, 2100 Copenhagen OE (DK); Wiken, Margareta, 113 51 Stockholm (SE)
(74) Representative: Salka, Jeffrey

(57) **Abstract**

The present invention provides a method for determining the in vitro potency of antibodies, in particular anti-Rhesus D positive antibodies. Such antibodies may e.g. be used in the prophylaxis of hemolytic disease of the newborn (HDN), treatment of idiopathic thrombocytopenic purpura (ITP) and prevention of sensitization to the Rhesus D antigen after mistransfusions of RhD(+) blood to RhD(-) individuals. The invention also provides a method for monitoring the release of label from a RhD(+) red blood cell, a method for determining the ability of an antibody to induce the release of label from a RhD(+) red blood cell, and a method for determining whether a manufactured anti-RhD antibody fulfils a predefined release criterion. The invention further provides a kit for measuring the potency of an antibody.

## Description

### Field of invention

The present invention relates to a method for determining the *in vitro* potency of antibodies, in particular anti-Rhesus D positive antibodies. Such antibodies may e.g. be used in the prophylaxis of hemolytic disease of the newborn (HDN), treatment of idiopathic thrombocytopenic purpura (ITP) and prevention of sensitization to the Rhesus D antigen after mistransfusions of RhD(+) blood to RhD(-) individuals. The invention also relates to a method for monitoring the release of label from a RhD(+) red blood cell comprising a detectable label and/or the amount of label taken up by phagocytotic cells, a method for determining the ability of an antibody to induce the release of label from a RhD(+) red blood cell comprising a detectable label and/or the ability of an antibody to induce the uptake of label from a RhD(+) red blood cell comprising a detectable label a by phagocytotic cells, and a method for determining whether a manufactured anti-RhD antibody fulfils a predefined release criterion. The invention further relates to a kit for measuring the potency of an antibody.

### Background of invention

The Rhesus blood group antigens are located on transmembrane erythrocyte proteins encompassing the so-called C, c, E, e and D antigens. Approximately 16% of the Caucasian population is Rhesus D negative (RhD(-)) due to an inherited polymorphism. In addition, multiple genetic and serological variants of RhD exist (divided inter alia into the categories II-VII as well as a large number of other categories) of which RHDVI is the most clinically relevant. Since category VI positive red blood cells (RBC) carry fewer of the various epitopes of the D protein than RBC of other categories, RhDVI(+) individuals may form alloantibodies against RBC from other RhD positive (RhD(+)) individuals (Issitt, P.D. and Anstee, D.J., 1998. The Rh Blood Group System, Montgomery Scientific Publications, Durham, North Carolina, pp. 315-423).

The RhD negative phenotype in itself is not associated with any medical conditions, but has important medical implications when a RhD(-) female carries a RhD(+) or RhDVI(+) fetus or a RhDVI(+) female carries a RhD(+) fetus. Fetomaternal RhD alloimmunization may then occur if fetal erythrocytes enter the maternal circulation, usually perinatally (during delivery), and thereby causes the induction of a maternal anti-RhD antibody response.

In subsequent pregnancies RhD-specific IgG-molecules from the mother will cross the placenta into the fetal circulation and mediate lysis of fetal erythrocytes, thereby causing Hemolytic Disease of Newborns (HDN). It has been estimated that on average 20% of RhD(-) women delivering a RhD(+) infant for the second time, and who are not protected appropriately with anti-D prophylaxis, will generate an anti-RhD antibody response. When untreated, approximately 30% of the newborn will have moderate anemia, jaundice, and hepatomegaly, and 20% develop severe anemia and hydrops fetalis, and severely affected newborns are at risk of neonatal death or permanent handicaps.

Polyclonal immunoglobulin preparations against RhD are used worldwide to prevent alloimmunization of pregnant RhD(-) and RhDVI(+) women, thereby preventing hemolytic disease of the newborn. Polyclonal immunoglobulin preparations against RhD (anti-D) are currently obtained by pooling of blood plasma obtained from donors who have become hyperimmune, either through natural RhD alloimmunization or through vaccination of RhD negative volunteer males with RhD positive erythrocytes. The efficacy of anti-RhD immunoglobulin preparations for prophylaxis of HDN is well established and has been in routine use for many years. As a result this severe disease has become a rarity.

In addition to the prophylaxis of HDN, anti-D immunoglobulin has also proven useful in the treatment of idiopathic thrombocytopenic purpura (ITP) (George, J.N., 2002. Blood Rev. 16, 37-38). ITP is a hematological disorder, where autoantibodies results in an accelerated platelet clearance in the spleen and liver. Symptoms are decreased platelet levels resulting in bruising and bleeding. In severe cases the spleen is removed. This is however, not possible in infants due to severe side effect, thus alternative treatments like anti-D immunoglobulin are needed. Further, anti-D immunoglobulin is used after mistransfusions of RhD(+) blood to RhD(-) recipients in order to prevent sensitization to the Rhesus D antigen.

WO 2006/007850 discloses a recombinant polyclonal anti-RhD antibody (anti-RhD rpAb) which can be used to replace the anti-D immunoglobulin currently on the market.

In order to evaluate the usefulness of antibodies, such as anti-RhD rpAb, for use for the above-mentioned purposes, it is convenient to employ an *in vitro* assay which may determine the ability of said antibodies to induce phagocytosis by phagocytotic cells and/or Antibody-Dependent Cellular Cytotoxicity, which may be an additional mechanism of action involved.

Previously, such an assay has been described in e.g. WO 2006/007850 A1, example 6. However, this assay is dependent upon the use of fresh blood from donors for the preparation of the effector cells, such as for example peripheral blood mononuclear cells (PBMCs) or monocytes as well as the RhD (+) red blood cell (RBC) target cells. Thus, there is a need to develop an assay, which is preferably less dependent upon the use of such fresh donor material.

### Summary of invention

The present invention inter alia provides new and improved methods for determining the potency of an antibody, in particular the ability of an antibody to induce the release of label from a RhD(+) red blood cell comprising a detectable label and/or the ability to induce the uptake of label from a RhD(+) red blood cell comprising a detectable label by phagocytotic cells. Preferably said antibody is an anti-RhD antibody. In particular, the use of phagocytotic cells having been cultured in vitro, e.g. phagocytotic cells from a cell line, has been introduced as a useful and superior alternative to the known use of Peripheral Blood Mononuclear Cells (PBMC).

The present invention provides methods for determining the potency of an antibody, said methods having a considerable lower variability of measurements as well as a greatly improved dynamic range of the measurements. It is considered that the provided methods will be of great value in reliably determining the potency and hence usefulness of antibodies.

Surprisingly, the present invention discloses that the substitution of PBMC by phagocytotic cells being non-adherent and having been cultured in vitro has dramatic positive effects on the dynamic range observed in the assay. Moreover, the reproducibility of the assay may also be positively affected by the new methods disclosed herein. Also, the substitution of the PBMC makes it possible to avoid the practical problems of obtaining effector cells from donors. Furthermore, such donated cells give rise to a large degree of unwanted variability in measurements as disclosed herein.

Furthermore, the use of non-adherent cells makes it possible to conduct the method without a number of extra time-consuming steps associated with the adherence of cells.

### Definitions

The term "antibody" describes a functional component of serum and is often referred to either as a collection of molecules (antibodies or immunoglobulin) or as one molecule (the antibody molecule or immunoglobulin molecule). An antibody molecule is capable of binding to or reacting with a specific antigenic determinant (the antigen or the antigenic epitope), which in turn may lead to induction of immunological effector mechanisms. An individual antibody molecule is usually regarded as monospecific, and a composition of antibody molecules may be monoclonal (i.e., consisting of identical antibody molecules) or polyclonal (i.e., consisting of different antibody molecules reacting with the same or different epitopes on the same antigen or even on distinct, different antigens). Each antibody molecule has a unique structure that enables it to bind specifically to its corresponding antigen, and all full-length antibody molecules have the same overall basic structure of two identical light chains and two identical heavy chains. Antibodies are also known collectively as immunoglobulins. The terms antibody or antibodies as used herein are also intended to include chimeric and single chain antibodies, as well as binding fragments of antibodies, such as Fab, Fab' or F(ab) 2 molecules, Fv fragments or scFv fragments or any other stable fragment, as well as full-length antibody molecules and multimeric forms such as dimeric IgA molecules or pentavalent IgM.

The term "Antibody-Dependent Cellular Cytotoxicity" as used herein denotes the process whereby effector cells of the immune system, such as monocytic cells, actively lyses target cells, e.g. red blood cells, which have been bound by an antibody.

The term "anti-RhD recombinant polyclonal antibody" or "anti-RhD rpAb" describes a composition of recombinantly produced diverse antibody molecules, where at least the majority, preferably at least 70%, more preferably at least 80%, even more preferably at least 90%, yet more preferably at least 95%, for example essentially all, such as all of the individual members are capable of binding to at least one epitope on the Rhesus D antigen. Preferably the composition may include different antibodies capable of binding different epitopes of the Rhesus D antigen. Preferably, the composition is produced from a single polyclonal manufacturing cell pool. The diversity of the polyclonal antibody is located in the variable regions (VH and VL regions), in particular in the CDR1, CDR2 and CDR3 regions.

The term "cell line" is used to denote an in vitro culture of cells isolated from an organism. The cell line may be primary or immortalised, preferably, they are immortalised and may be kept in any suitable medium. The immortalisation may have brought about by the insertion of an immortalisation gene or may be spontaneous as can be seen e.g. for cancer cells. The cells in a cell line may in one preferred embodiment originate from one and the same cell, in which case the cell line is referred to as being "a monoclonal cell line". Alternatively, in other embodiments of the invention the cells in the cell line may originate from a plurality of different cells in which case it is referred to as a "polyclonal cell line". The cells in a cell line may in a preferred embodiment be homogenous. In another embodiment the cells of a cell line may be heterogeneous depending on their origin and possible differentiation.

The term "detectable label" as used herein means that any given label that may be detected by at least one and possibly a plurality of detection methods, for example by measuring emission of electromagnetic radiation or particles, for instance measuring radioactive decay of radioactive isotope labels, measuring the emission of light from different types of fluorescent labels, measuring spectrophotometric properties of dyes, colours, and naturally coloured molecules such as haemoglobin, and measuring the presence of heavy metal labels. A detectable label may either be a supplied label, i.e. a label which is not inherently present in the labelled entity, or an inherent label, i.e. a label which is inherently present in the labelled entity.

The terms "a distinct member of the anti-RhD rpAb" denotes an individual antibody molecule of the recombinant polyclonal antibody composition, comprising one or more stretches within the variable regions, which are characterized by differences in the amino acid sequence compared to the other individual members of the polyclonal protein. These stretches are in particular located in the CDR1, CDR2 and CDR3 regions.

The term "epitope" means a protein determinant which an antibody is capable of specifically binding to. Epitopes usually consist of chemically active surface groupings of molecules such as amino acids or sugar residues and usually have specific three dimensional structural characteristics, as well as specific charge characteristics. Conformational and non conformational epitopes are distinguished in that the binding to the former but not the latter is lost in the presence of denaturing solvents.

The term "full-length antibody" as used herein refers to a full-length antibody consisting of two heavy chains and two light chains. The term is not intended to be limited to naturally occurring antibodies, but includes all full-length antibodies consisting of two heavy and two light chains. Each heavy chain comprises a constant region, in general containing several constant domains and a variable domain. The light chains likewise comprise a constant domain and a variable domain. Full-length antibodies exist in a number of classes. In mammals the five antibody classes are known as IgA, IgD, IgE, IgG, and IgM. Antibodies are Y-shaped proteins. The two arms of the Y contain the sites that bind antigen and, therefore, recognize specific foreign objects. This region of the antibody is called the Fab (fragment, antigen binding) region. It is composed of one constant and one variable domain from each heavy and light chain of the antibody. The base of the Y plays a role in modulating immune cell activity. This region is called the Fc (Fragment, crystallisable) region, and is composed of two heavy chains that contribute two or three constant domains depending on the class of the antibody.

The term "immunoglobulin" herein is used as a collective designation of the mixture of antibodies found in blood, plasma or serum.

The term "inherent label" refers to a label which has not been synthetically incorporated but is naturally present in a given cell or molecule. Such an inherent label may e.g. be the haemoglobin in a red blood cell.

The term "International Reference Preparation" as used herein refers to one of the World Health Organization (WHO) International Biological Reference Preparations as held and distributed by the WHO International Laboratories for Biological Standards. Among these Reference Preparations are the "Anti-D (anti-Rh0) blood-grouping reagent, complete, lyophilized (1 in 3 dilution recommended)" monoclonal human IgM having code no. 99/836 and WHO/BS DOCUMENT no. 04.2000 Rev. 1, the "Anti-D (anti-Rh0) incomplete blood-typing serum, human, lyophilized (32 IU/ampoule)" human serum having code no. W1006 and WHO/BS DOCUMENT no. 66.810, and the "Anti-D immunoglobulin, human, lyophilized (285 IU (57 µg)/ampoule)" human immunoglobulin having code no. 01/572 and WHO/BS DOCUMENT no. 03.1962.

The term "non-adherent" as used herein means cells, which in the particular state they are in do not adhere to a solid surface, such as another cell or a surface, for example the bottom of a culture vial or microtiter plate. Cellular adhesion is regulated by specific adhesion molecules that interact with molecules on an opposing cell or a surface. Accordingly, non-adherent cells comprise only to a limited extent, or preferably not at all specific adhesion molecules, which interact with molecules on an opposing cell or surface. In general, it will be possible to gently remove non-adherent cells from any given solid surface. Non-adherent cells may alter their properties and become adherent for example after differentiation.

The term "phagocytotic" refers to the capability of a cell to phagocytise, i.e. "engulfe" or "internalise" material which is present outside said cell. For instance, cells bound by antibodies may be phagocytised by a number of different cells of the human immune system.

The term "polyclonal antibody" describes a composition of different (diverse) antibody molecules, which is capable of binding to or reacting with several different specific antigenic determinants on the same or on different antigens. Usually, the variability of a polyclonal antibody is located in the so-called variable regions of the polyclonal antibody, in particular in the CDR regions. When stating that a member of a polyclonal antibody binds to an antigen, it is herein meant a binding having a binding constant that is below 1 mM, preferably below 100 nM, even more preferred below 10 nM.

The term "potency" as used herein means a measure of the ability of an antibody, preferably an anti-RhD antibody to cause Antibody-Dependent Cellular Cytotoxicity (ADCC) and/or a measure of the ability of an antibody, preferably an anti-RhD antibody to cause phagocytosis by a phagocytotic cell of a cell to which the antibody is bound. The term potency may refer to either of the two above-mentioned measures or optionally to a combination of the two.

The term "predefined release criterion" refers to one or more values of measurable characteristics of a product. Such values may for instance be measurements of the ability of an antibody to cause Antibody-Dependent Cellular Cytotoxicity or phagocytosis. Such values may be used to determine whether a given batch is suited for release (e.g. for sale or for medical use), e.g. by comparison to reference values.

The term "recombinant antibody" is used to describe an antibody molecule or several molecules that is/are expressed from a cell or cell line transfected with an expression vector comprising the coding sequence of the protein which is not naturally associated with the cell. If the antibody molecules are diverse or different, the term "recombinant polyclonal antibody" applies in accordance with the definition of a polyclonal antibody.

### Abbreviations

Ab= antibody. ADCC = Antibody-Dependent Cellular Cytotoxicity. Anti-RhD rpAb= anti-Rhesus D recombinant polyclonal antibody. HDN= hemolytic disease of the newborn. ITP= idiopathic thrombocytopenic purpura. mAb= monoclonal antibody. PBMC = Peripheral Blood Mononuclear Cells. RBC=red blood cells. RhD= Rhesus D. RhD(-)= Rhesus D negative. RhD(+)= Rhesus D positive. RhDVI= Rhesus D category VI antigen. Anti-D=polyclonal immunoglobulin preparation against RhD prepared from plasma of hyperimmune donors.

### Description of Drawings

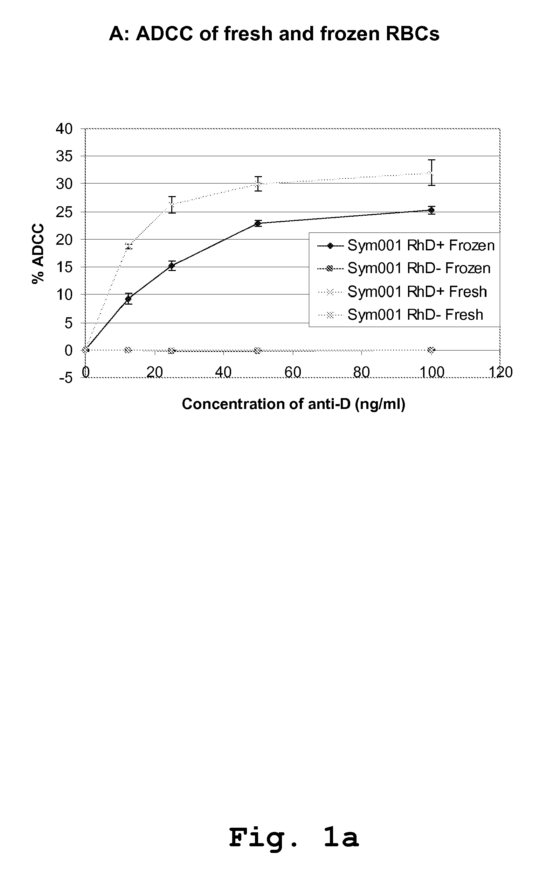

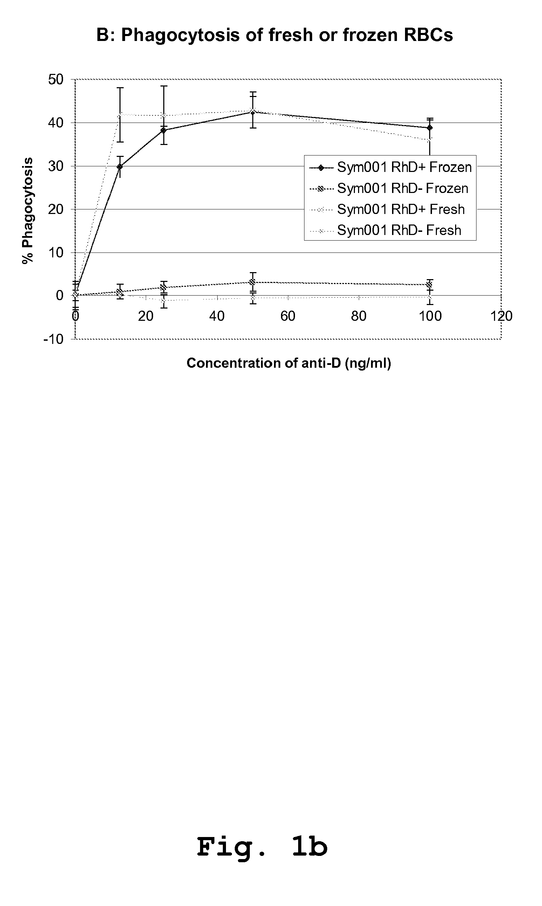

Fig. 1 a: Effect of using fresh or frozen RBC in the PBMC based ADCC assay. The percentage of PBMC mediated ADCC of RhD positive or negative RBC as a function of concentration of anti-RhD antibodies from Sym001. The individual measurement is based on triplicates. The standard deviations are indicated by bars.

Fig. 1b: Effect of using fresh or frozen RBC in the PBMC based phagocytosis assay. The percentage of PBMC mediated phagocytosis of RhD positive or negative RBC as a function of concentration of anti-RhD antibodies from Sym001. The individual measurement is based on triplicates. The standard deviations are indicated by bars.

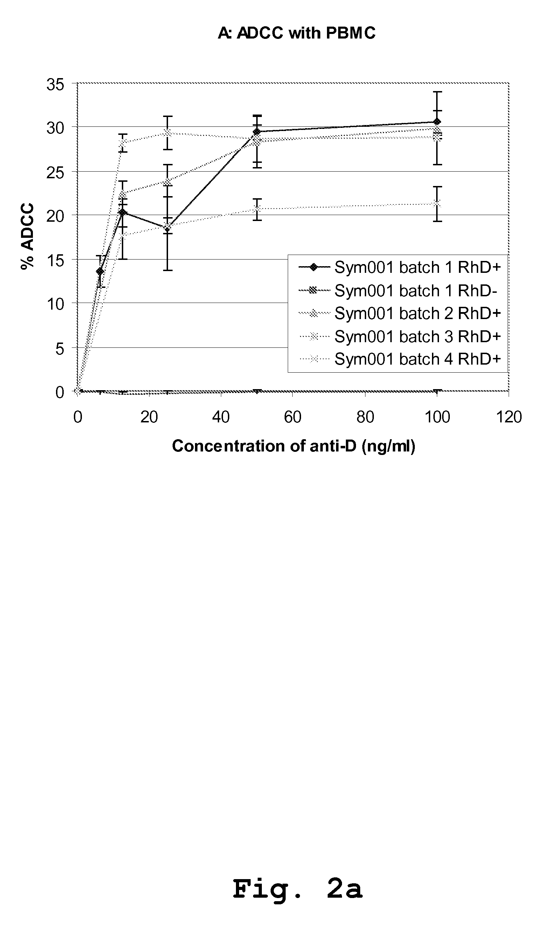

Fig. 2a: Batch-to-batch variability of ADCC using the PBMC based assay. The percentage of PBMC mediated ADCC of RhD positive or negative RBC as a function of concentration of anti-RhD antibodies in 4 different batches of Sym001. The individual measurement is based on triplicates. The standard deviations are indicated by bars.

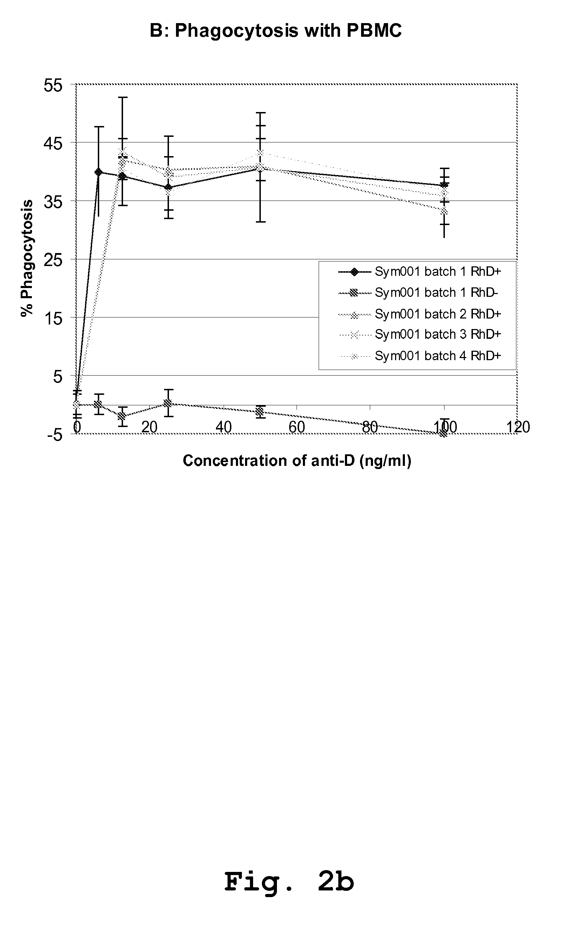

Fig. 2b: Batch-to-batch variability of phagocytosis using the PBMC based assay. The percentage of PBMC mediated phagocytosis of RhD positive or negative RBC as a function of concentration of anti-RhD antibodies in 4 different batches of Sym001. The individual measurement is based on triplicates. The standard deviations are indicated by bars.

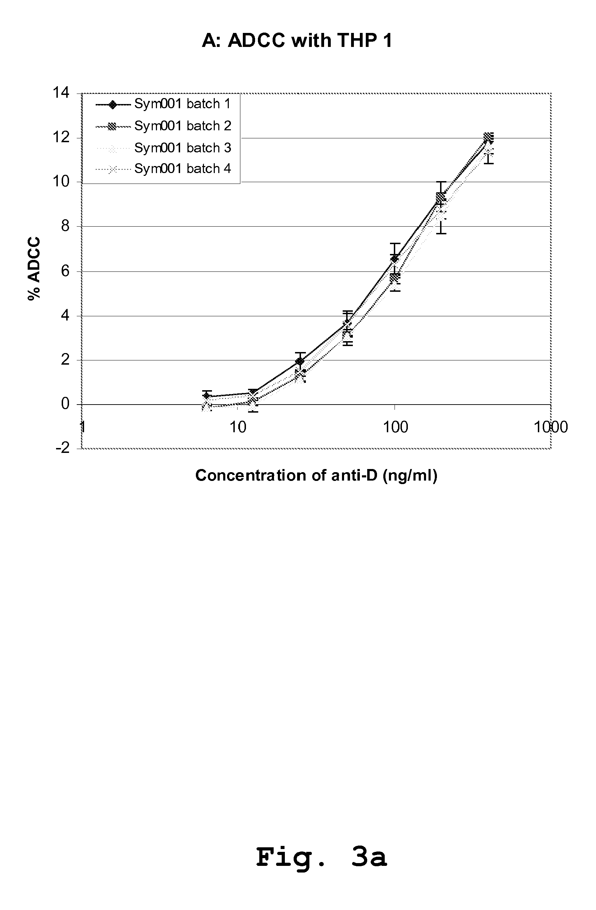

Fig. 3a: Batch-to-batch variability of ADCC using the THP-1 based assay. The percentage of THP-1 cell mediated ADCC of RhD positive RBC as a function of concentration of anti-RhD antibodies in 4 different batches of Sym001. The individual measurement is based on triplicates. The standard deviations are indicated by bars.

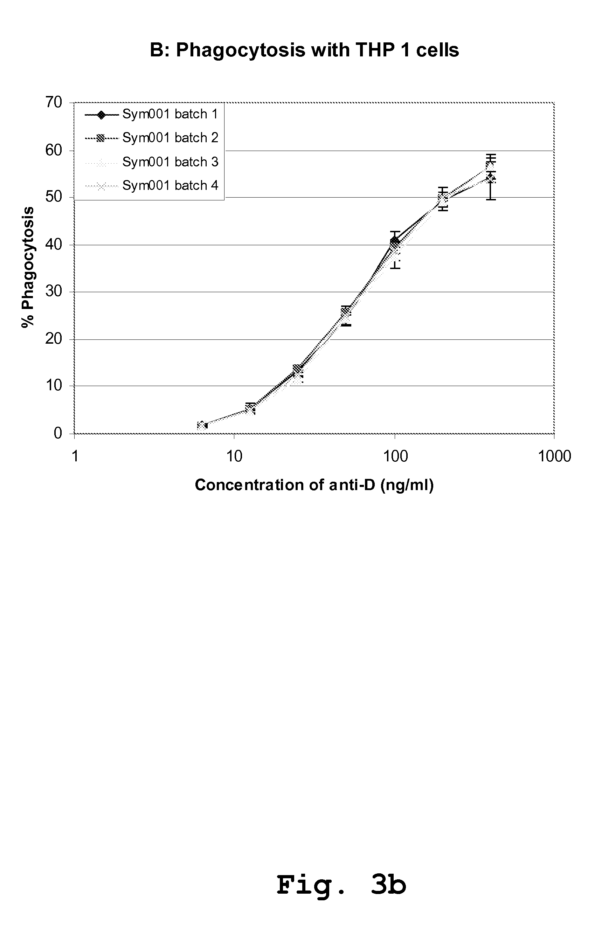

Fig. 3b: Batch-to-batch variability on phagocytosis using the THP-1 based assay. The percentage of THP-1 cell mediated phagocytosis of RhD positive RBC as a function of concentration of anti-RhD antibodies in 4 different batches of Sym001. The individual measurement is based on triplicates. The standard deviations are indicated by bars.

### Detailed description of the invention

The present invention provides a method for determining the potency (preferably the the ability to induce the release of label from a RhD(+) red blood cell comprising a detectable label and/or the ability to induce the uptake of label from a RhD(+) red blood cell comprising a detectable label by phagocytotic cells) of an antibody (preferably an anti-RhD antibody), said method comprising the steps of:
a) providing RhD(+) red blood cells comprising a detectable label,
b) providing at least one anti-RhD antibody the potency of which it is desirable to determine,
c) contacting said RhD(+) red blood cells with said at least one anti-RhD antibody, thereby obtaining a mixture of said RhD(+) red blood cells and said at least one anti-RhD antibody,
d) providing phagocytotic cells, said phagocytotic cells having been cultured in vitro,
e) contacting said mixture of said RhD(+) red blood cells and said at least one anti-RhD antibody with said phagocytotic cells, and
f) measuring the amount of label released from said RhD(+) red blood cells and/or the amount of label taken up by said phagocytotic cells,
wherein said amount of label released and/or said amount of label taken up is a measure of the potency of said at least one anti-RhD antibody.

In specific embodiments of the invention the methods may include the further steps of:
g) providing RhD(-) red blood cells comprising a detectable label,
h) providing at least one anti-RhD antibody the potency of which it is desirable to determine,
i) contacting said RhD(-) red blood cells with said at least one anti-RhD antibody, thereby obtaining a mixture of said RhD(-) red blood cells and said at least one anti-RhD antibody,
j) providing phagocytotic cells, said phagocytotic cells having been cultured in vitro,
k) contacting said mixture of said RhD(-) red blood cells and said at least one anti-RhD antibody with said phagocytotic cells, and
I) measuring the amount of label released from said RhD(-) red blood cells and/or the amount of label taken up by said phagocytotic cells.
Such further method steps may serve as a control measurement, since no specific binding will take place between RhD(-) red blood cells and anti-RhD antibodies.

When conducting the above-mentioned method, RhD positive red blood cells comprising some sort of detectable label are provided. The label may be contained within said red blood cells, for example within the cytoplasm of the RBC. The label may also be located within or at the membrane of the RBC. The RhD positive red blood cells may comprise any serological or genetic variant of RhD (divided into the categories II-VII). The RhD positive red blood cells may originate from a single donor or from a plurality of different donors. The donors are preferably humans. The donors may be either healthy or diseased individuals. The RBC may optionally be obtained from a transgenic organism or a plurality of transgenic organisms.

The label may be any suitable detectable label, which can later in the procedure be detected. The label may be either supplied, i.e. not inherently present in the labelled entity, or inherent, i.e. inherently present in the labelled entity. It is preferred that the label is supplied. Preferred supplied labels include radioactive labels, colours, dyes, fluorescent labels, fluorochromes, fluorophores, and heavy metals. A preferred inherent label is haemoglobin. Suitable fluorescent labels include e.g. the group of Alexa Flour fluorophores as well as Texas Red and Oregon Green all marketed by Molecular Probes (Invitrogen). A large number of additional different suitable labels to be used with the present invention may also be found in the catalogue of labels provided by Molecular Probes (Invitrogen). Preferably, the label may be a radioactive isotope, which may be detected by means of e.g. a scintillation counter or radiography. Radioactive labels include Sulphur-35, Phosphorus-32, Phosphorus-33, Chromium-51, and lodine-125. Most preferably the radioactive label used is Chromium-51.

The red blood cells are brought into contact with at least one anti-RhD antibody. Said anti-RhD antibody is preferably an antibody which is capable of specifically binding to the RhD antigen. The RhD antigen is preferably RhD antigen as described in the online database of "The Rhesus Site" (http://www.uni-ulm.de/-wflegel/RH/) or in "Human Blood Groups" (Blackwell Science, pp. 195-274), preferably as described in the online database of "The Rhesus Site" (http://www.uni-ulm.de/-wflegel/RH/) on 4 October 2007 or in "Human Blood Groups" (Blackwell Science, pp. 195-274), more preferably in "Human Blood Groups" (Blackwell Science, pp. 195-274). "Specifically binding" in the context of the present invention means that the antibody is capable of binding an epitope of RhD antigen with a binding constant that is below 1 mM, preferably below 100 nM, even more preferred below 10 nM. An antibody specifically binding RhD antigen may in some embodiments also bind other antigens, however preferably with a lower affinity. Typically, a large number of individual, either identical or different, antibodies in solution are used. For example the antibody may be an anti-RhD recombinant polyclonal antibody as defined herein above.

The antibody may be any antibody known in the art, for example a polyclonal or a monoclonal antibody derived from a mammal or a synthetic antibody, such as a single chain antibody or hybrids comprising antibody fragments. The antibodies may preferably be full-length antibodies. The antibodies may also be isolated immunoglobulins or existing compositions of antibodies, such as the commercially available pharmaceutical anti-D immunoglobulin compositions, such as those marketed as Rhophylac® and WinRho®. Furthermore, the antibody may be mixtures of monoclonal antibodies or recombinant polyclonal antibodies. Recombinant polyclonal antibodies may for example be prepared as described in WO 2006/007850 A1, p. 14, line 5 to p. 54, line 10 as well as the figures 3-6. WO 2006/007850 in its entirety is hereby incorporated by reference. In addition functional equivalents of antibodies may be antibody fragments, in particular epitope binding fragments. Furthermore, antibodies or functional equivalent thereof may be small molecule mimic, mimicking an antibody.

The antibodies according to the present invention may also be recombinant antibodies. Recombinant antibodies are antibodies or fragments thereof or functional equivalents thereof produced using recombinant technology. For example recombinant antibodies may be produced using a synthetic library or by phage display. Recombinant antibodies may be produced according to any conventional method for example the methods outlined in "Recombinant Antibodies", Frank Breitling, Stefan Dübel, Jossey-Bass, September 1999.

The antibodies according to the present invention may also be bispecific antibodies, i.e. antibodies specifically recognising two different epitopes. Bispecific antibodies may in general be prepared starting from monoclonal antibodies, or from recombinant antibodies, for example by fusing two hybridoma's in order to combine their specificity, by Chemical crosslinking or using recombinant technologies. Antibodies according to the present invention may also be tri-specific antibodies.

Usually, the step of contacting the red blood cells with the antibodies is carried out by adding a solution of antibodies to the medium in which the red blood cells are kept. This may for instance conveniently be done in microtiter plates. When the RhD positive antibodies come into contact with the RhD positive red blood cells, some of the antibodies will, depending on exact specificities and concentration, bind to available antigenic epitopes of the RhD antigen located on the red blood cells.

Phagocytotic cells having been cultured *in vitro* are then added to the mixture of red blood cells and antibody. Preferably, "cultured *in vitro"* involves expanding a population of cells through repeated cell divisions to obtain a larger population of genetically uniform, preferably identical cells. Expansion may occur as a result of immortalisation of the cell line or be caused by the addition of growth factors to the cells, depending on the cells. Methods for culturing cells for use according to the invention are well known to the person skilled in the art.

These phagocytotic cells, also commonly referred to as "effector cells", will recognize red blood cells bound by antibody and subsequently phagocytise such cells, i.e. internalise the cells including the label. Alternatively, the phagocytotic cells may employ the mechanism of ADCC to lyse the red blood cells bound by antibody, thus causing the release of the contents of the red blood cells, including label, to the surrounding medium.

The phagocytotic cells having been cultured in vitro may be of any suitable type, but preferably they are non-adherent, phagocytic cells. In particular, the phagocytotic cells may be a mixture of a plurality of different phagocytotic cells, preferably different non-adherent phagocytic cells. In another preferred embodiment the phagocytotic cells are all the same kind of phagocytotic cell. Particularly, the phagocytotic cells may be chosen from the group consisting of: monocytes, neutrophils, macrophages, granulocytes, and dendritic cells. The phagocytotic cells having been cultured in vitro may originate from a cell line of phagocytotic cells. Preferably the phagocytotic cells having been cultured in vitro are monocytes. More preferably the phagocytotic cells having been cultured in vitro are monocytes originating from a monocytic cell line, for example a monoclonal cell line. Examples of monocytic cell lines can be found in Drexler, 2000 ("Monocytic Cell Lines", pp 237-257, Chapter 7 in Human Cell Culture Vol III, Masters & Palsson (eds), Kluwer Academic Publishers, Netherlands, 2000). In one embodiment, the cell line is selected from the group consisting of monocytic cell lines AML-1, AML-193, CTV-1, DOP-M1, FLG29.1, IMS-M1, KBM-3, KBM-5, KP-1, KP-MO-TS, ME-1, ML-2, MOLM-13, Mono Mac 6, MUTZ-3, MV4-11, NOMO-1, OCI/AML-2, OMA-AML-1, P31/Fujioka, P39/Tsugane, PLB-985, RC-2A, RWLeu-4, SCC-3, SKM-1, THP-1, TK-1, U-937, UG3, and YK-M2. The majority of these are available from one or more of the depositary institutions, ATCC (P.O. Box 1549, Manassas, VA20108, USA), DSMZ (DSMZ - Inhoffenstraße 7 B, 38124 Braunschweig, GERMANY), JCRB (Japanese Collection of Research Bioresources, available through Health Science Research Resources Bank(HSRRB) Cell line distribution center. Rinkuminamihama 2-11, Sennan-shi, Osaka 590-0535, Japan), or from Dr. Drexler (c/o DSMZ, Department of Human and Animal Cell Cultures) as indicated in the above-referenced chapter. Monocytic cell lines that are available from at least one of these sources include AML-193, CTV-1, ME-1, ML-2, MOLM-13, Mono Mac 6, MUTZ-3, MV4-11, NOMO-1, OCI/AML-2, P31/Fujioka, P39/Tsugane, PLB-985, SCC-3, SKM-1, THP-1, and U-937. Still more preferably the monocytic cell line is selected from the group consisting of cell lines that are known to be phagocytosis positive (see Table V of Drexler, 2000). Phagocytosis positive cell lines include, according to Drexler, KP-1, KP-MO-TS, ME-1, Mono Mac 6, NOMO-1, OMA-AML-1, P31/Fujioka, P39/Tsugane, SCC-3, THP-1, U-937, and YK-M2.

Most preferably the phagocytotic cells having been cultured *in vitro* are monocytes originating from at least one of the monocytic cell lines ME-1, Mono Mac 6, NOMO-1, P31/Fujioka, P39/Tsugane, SCC-3, THP-1, and U-937, even more preferably from THP-1 or U937, yet more preferably THP-1.

It is preferred that the phagocytotic cells mentioned herein above are non-adherent, in particular it is preferred that at the particular time, when they are used with the methods of the present invention, then they are non-adherent. In one embodiment, the cells are kept in suspension. Accordingly, it is preferred that the phagocytotic cells have not differentiated into adherent cells prior to or simultaneously with their use in the methods of the invention. In addition it is preferred that the phagocytotic cells are kept *in vitro* at conditions, which do not result in differentiation into adherent cells. This does not exclude the possibility that cytokines or other stimulating agents are applied to the cells to stimulate them prior to use in the assays of the invention.

In one particular embodiment of the invention, the effector cells may be both cytolytic and phagocytotic, as one particular cell may act in different ways (phagocytosis or ADCC) depending on conditions used.

It is afterwards possible to separately measure both the label taken up by the phagocytotic cells as well as the label released to the medium as a result of the ADCC mechanism. The exact measuring techniques employed obviously depend on the label used. Such measuring techniques for a large number of different labels are well known to the skilled person. In general, the measurement may be accomplished by measuring emission of electromagnetic radiation or particles such as measuring fluorescence, radioactive decay or counting cells from which such emission of radiation or particles occurs. For instance, if the compound for example is a fluorescent compound, the level of said compound may be determined by determining the fluorescence properties. This may be done by any suitable means, for example by the aid of a fluorescence microscope, a flow cytometer (for example a FACS (Fluorescence Activated Cell Sorter)), a fluorescence plate-reader or a fluorescence spectrometer. When employing a FACS to measure the amount of label taken up by the phagocytotic cells, the actual value measured is the number of cells, which have phagocytised labelled material. This is thus an indirect measure of the total amount of label taken up. Alternatively, if the label is a radioactive label the measurement may be accomplished by a number of techniques such as scintillation counting or radiography.

The values obtained through such measurement of label taken up and label released can be used as a measure of the potency of the antibody or antibody mixture used. Commonly, the values obtained are compared to one or more standard values obtained by testing well known antibodies or mixtures of antibodies in the same assay. Preferably, the potency is compared to the potency of International Reference Preparations, more preferably to Reference Preparations having code no. 99/836, code no. W1006 or code no. 01/572 (see more details herein above under the definition of "International Reference Preparations "). Alternatively, the potency is compared to the potency of an in house reference preparation that has been calibrated against said International Reference Preparations. The predefined release criterion may be any predefined release criterion, however in one embodiment it is at least 50%, more preferably at least 60%, even more preferably at least 70%, yet more preferably at least 80%, even more preferably at least 90%, for example at least 95%, such as at least 100%, for example at least 120%, such as at least 140%, for example at least 150%, such as at least 170%, for example at least 200%, such as at least 250% of the potency of the International Reference Preparations or a calibrated in house reference preparation mentioned above.

The methods of the invention have the advantages of resulting in measurements with a considerably lower variability. Interestingly, the methods of the invention also have a significantly higher dynamic range than the known methods within the field. In this context, the dynamic range is defined as the range of antibody concentrations for which there is a linear or substantially linear correlation to the specific parameter measured. The measured parameter in the present context may preferably be the amount of label released from labelled RhD(+) RBC which corresponds to the amount of ADCC having taken place, e.g. the percentage of ADCC observed as compared to a given control. The measured parameter in the present context may also preferably be the amount of label from labelled RhD(+) RBC taken up by phagocytotic cells, which corresponds to the amount of phagocytosis having taken place, e.g. the percentage of phagocytosis observed as compared to a given control. The dynamic range may preferably be at least one decade of antibody concentrations, i.e. from a given first concentration of antibody to a second concentration of antibody being either 10 times higher or 10 times lower than the first concentration, e.g. from 1 ng/ml to 10 ng/ml. More preferably the dynamic range may be at least 2 decades of antibody concentration, e.g. from 1 ng/ml to 100 ng/ml. More preferably the dynamic range may be at least 3 decades of antibody concentration, e.g. from 1 ng/ml to 1000 ng/ml. More preferably the dynamic range may be at least 4 decades of antibody concentration, e.g. from 0,1 ng/ml to 1000 ng/ml. More preferably the dynamic range may be at least 5 decades of antibody concentration, e.g. from 0,01 ng/ml to 1000 ng/ml.

The effects on dynamic range are e.g. clear when comparing Fig. 2a to Fig. 3a and Fig. 2b to Fig. 3b. In Fig. 2a and Fig. 2b, where PBMC are used, the dynamic range is only within the range of 0 to approximately 20 ng/ml concentration of antibody. In comparison, the dynamic range in Fig. 3a where cells from the monocytic cell line THP-1 are used in an assay for ADCC, is from about 10 to at least 300 ng/mL concentration of antibody. In Fig. 3b where cells from the monocytic cell line THP-1 are used in an assay for phagocytosis, the dynamic range is from about 10 to about 300 ng/ml concentration of antibody.

The present invention also provides a method for monitoring the release of label from a RhD(+) red blood cell and/or the amount of label taken up by phagocytotic cells, said phagocytotic cells being non-adherent and said phagocytotic cells having been cultured in vitro, said method comprising the steps of:
a) providing RhD(+) red blood cells comprising a detectable label,
b) providing at least one anti-RhD antibody,
c) contacting said RhD(+) red blood cells with said at least one anti-RhD antibody, thereby obtaining a mixture of said RhD(+) red blood cells and said at least one anti-RhD antibody,
d) providing phagocytotic cells, said phagocytotic cells being non-adherent and said phagocytotic cells having been cultured in vitro,
e) contacting said mixture of said RhD(+) red blood cells and said at least one anti-RhD antibody with said phagocytotic cells, and
f) measuring the amount of label released from said RhD(+) red blood cells and/or the amount of label taken up by said phagocytotic cells.

The invention further provides a method for determining the ability of an antibody to induce the release of label from a RhD(+) red blood cell comprising a detectable label and/or the ability to induce the uptake of label from a RhD(+) red blood cell comprising a detectable label a by phagocytotic cells, said phagocytotic cells being non-adherent and said phagocytotic cells having been cultured in vitro, said method comprising the steps of:
a) providing RhD(+) red blood cells comprising a detectable label,
b) providing at least one anti-RhD antibody,
c) contacting said RhD(+) red blood cells with said at least one anti-RhD antibody, thereby obtaining a mixture of said RhD(+) red blood cells and said at least one anti-RhD antibody,
d) providing phagocytotic cells, said phagocytotic cells being non-adherent and said phagocytotic cells having been cultured in vitro,
e) contacting said mixture of said RhD(+) red blood cells and said at least one anti-RhD antibody with said phagocytotic cells, and
f) measuring the amount of label released from said RhD(+) red blood cells and/or the amount of label taken up by said phagocytotic cells.

The invention further provides a method for determining whether a manufactured anti-RhD antibody fulfils a predefined release criterion, said method comprising the steps of:
a) providing RhD(+) red blood cells comprising a detectable label,
b) providing at least one anti-RhD antibody the potency of which it is desirable to determine,
c) contacting said RhD(+) red blood cells with said at least one anti-RhD antibody, thereby obtaining a mixture of said RhD(+) red blood cells and said at least one anti-RhD antibody,
d) providing phagocytotic cells, said phagocytotic cells being non-adherent and said phagocytotic cells having been cultured in vitro,
e) contacting said mixture of said RhD(+) red blood cells and said at least one anti-RhD antibody with said phagocytotic cells, and
f) measuring the amount of label released from said RhD(+) red blood cells and/or the amount of label taken up by said phagocytotic cells,
   wherein said amount of label released and/or said amount of label taken up can be compared to said predetermined release criterion.

In particular embodiments of the invention the RhD(+) red blood cells may originate from a single donor or alternatively from a plurality of different donors. The RhD(+) red blood cells may either have been frozen prior to use or be "fresh", i.e. not have been frozen prior to use.

In further embodiments of the invention the detectable label may be chosen from the group consisting of: a radioactive label, a colour, a dye, a fluorescent label, a fluorochrome, a fluorophore, a heavy metal, haemoglobin, and an inherent label. Particularly, the detectable label may be Chromium-51 (Cr-51).

In further embodiments of the invention the anti-RhD antibody may be chosen from the group consisting of: monoclonal antibodies, polyclonal antibodies, recombinant antibodies, isolated immunoglobulins, binding fragments of antibodies, chimeric antibodies, and single chain antibodies. Particularly, the anti-RhD antibody may be a polyclonal recombinant antibody or a mixture of a plurality of different monoclonal antibodies.

Since a large number of different antigenic epitopes exist on the RhD antigen, a similar large diversity of anti-RhD antibodies exist. Specific examples of anti-RhD antibodies testable with the methods of the present invention include the recombinant polyclonal antibodies as well as the individual antibodies disclosed in the earlier publication WO 2006/007850 A1, p. 14, line 5 to p. 54, line 10 as well as the figures 3-6. Further examples include a large number of monoclonal antibodies (mAbs) with Rhesus D antigen binding specificity that have been made using phage display (WO 97/49809 and Siegel, D.L et al. 2002. Transfus. Clin. Biol. 9, 83-97).

In further embodiments of the invention the phagocytotic cells may be a mixture of a plurality of different phagocytotic cells. Preferably, the phagocytotic cells may be chosen from the group consisting of: monocytes, neutrophils, macrophages, granulocytes, and dendritic cells. More preferably the phagocytotic cells may be obtained from a cell line, which is either spontaneously immortal, or has been immortalised. Suitable phagocytotic cell lines include, KP-1, KP-MO-TS, ME-1, Mono Mac 6, NOMO-1, OMA-AML-1, P31/Fujioka, P39/Tsugane, SCC-3, THP-1, U-937, and YK-M2.

Most preferably the phagocytotic cells having been cultured *in vitro* are monocytes originating from at least one of the monocytic cell lines ME-1, Mono Mac 6, NOMO-1, P31/Fujioka, P39/Tsugane, SCC-3, THP-1, and U-937, even more preferably from THP-1 or U937, yet more preferably THP-1.

The cell line ME-1 is available from DSMZ and has accession number ACC 537. The cell line Mono Mac 6 is available from DSMZ and has accession number ACC 124. The cell line NOMO-1 is available from DSMZ and has accession number ACC 542. The cell line P31/Fujioka is available from JCRB and has accession number JCRB0091. The cell line P39/Tsugane is available from JCRB and has accession number JCRB0092. The cell line SCC-3 is available from JCRB and has accession number JCRB0115.

The cell line THP-1 is available from LGC Promochem (LGC Promochem, Queens Road, Teddington, Middlesex TW11 OLY, UK) or from ATCC (P.O. Box 1549, Manassas, VA20108, USA) and has ATCC no. TIB 202. THP-1 is a monocytic cell line isolated from peripheral blood. The cell line is further described in e.g. Tsuchiya et al. (Cancer Res. 1982 Apr;42(4):1530-6).

The cell line U937 is available from LGC Promochem-ATCC and has ATCC no. CRL-1593.2. U937 is a monocytic cell line and further described in e.g. Ralph et al. (J. Exp. Med. 143: 1528-1533, 1976).

In further embodiments of the invention the measuring of the amount of label released from said RhD(+) red blood cells and/or the amount of label taken up by said phagocytotic cells may be carried out over at least 1 decade of antibody concentration, optionally at least 2 decades of antibody concentration, optionally at least 3 decades of antibody concentration, optionally at least 4 decades of antibody concentration, optionally at least 5 decades of antibody concentration. A "decade" in this context means a range from a first concentration to a second concentration, where the second concentration is either 10 times larger or 10 time smaller than the first concentration. For example, a measurement over 2 decades could be a measurement covering antibody concentrations from 10 ng/ml to 1000 ng/ml, whereas a measurement over 3 decades could be a measurement covering antibody concentrations from 1 ng/ml to 1000 ng/ml.

The present invention further relates to a kit for measuring the potency of an antibody, said kit comprising:
a) a detectable label for labelling RhD(+) red blood cells,
b) phagocytotic cells, said phagocytotic cells being suitable for *in vitro* culture, and
c) a standard anti-RhD antibody composition with a pre-determined potency.

Such a kit could inter alia be suitable for performing the methods of the present invention.

In particular embodiments of the invention, the detectable label comprised in the kit may be chosen from the group consisting of: a radioactive label, a colour, a dye, a fluorescent label, a fluorochrome, a fluorophore, a heavy metal, haemoglobin, and an inherent label. Particularly, the detectable label may be Chromium-51.

In further embodiments of the invention the phagocytotic cells comprised in the kit may be a mixture of a plurality of different phagocytotic cells. Particularly, the phagocytotic cells may be chosen from the group consisting of: monocytes, neutrophils, macrophages, granulocytes, and dendritic cells. Furthermore, the phagocytotic cells may be obtained from a cell line. The cell line may be chosen from the group consisting of: KP-1, KP-MO-TS, ME-1, Mono Mac 6, NOMO-1, OMA-AML-1, P31/Fujioka, P39/Tsugane, SCC-3, THP-1, U-937, and YK-M2. Most preferably the phagocytotic cells having been cultured *in vitro* are monocytes originating from at least one of the monocytic cell lines ME-1, Mono Mac 6, NOMO-1, P31/Fujioka, P39/Tsugane, SCC-3, THP-1, and U-937, even more preferably from THP-1 or U937, yet more preferably THP-1.

In a further embodiment of the invention the standard anti-RhD antibody composition comprised in the kit may be a standard preparation that has been calibrated against an International Reference Preparation. Such International Reference Preparations are held and distributed by the WHO International Laboratories for Biological Standards and are further described and exemplified herein above.

### Examples

### Example 1:

The present example demonstrates that different batches of an anti-RhD recombinant polyclonal antibody (rpAb) with 25 individual members show comparable biological activity with respect to the relevant effector mechanisms: Antibody-dependent cellular cytotoxicity (ADCC) and phagocytosis. Furthermore it shows that data generated with the monocytic cell line THP-1 has a larger dynamic range and less standard deviation.

### Preparation of red blood cells - frozen

Red blood cells (RBC) from whole blood obtained from healthy donors after informed consent at the Blood Bank, Aalborg Hospital, DK, were frozen by the high glycerol technique (38%) and stored at -80°C. The erythrocytes were thawed in 12% NaCl (Merck) and citrate-manitol (LAB20910.0500, Bie & Berntsen) was added after 3 min. The cells were washed 3 times in PBS (Invitrogen, CA, US) and stored at 4°C as a 3% solution in ID-Cellstab (DiaMed, Switzerland).

### Preparation of red blood cells - fresh

Red blood cells (RBC) were prepared from whole blood obtained from healthy donors after informed consent at the Blood Bank, Aalborg Hospital, DK, by washing the blood three times in PBS (Gibco, Invitrogen, United Kingdom) containing 1 % bovine serum albumin (BSA, Sigma-Aldrich, Germany). The erythrocytes were resuspended and stored at 4°C as a 10% solution in ID-Cellstab (DiaMed, Switzerland).

### Preparation of PBMC

Buffy coats containing blood from healthy donors were obtained from the Blood Bank at the National Hospital, Copenhagen, Denmark and peripheral blood mononuclear cells (PBMC) were purified on Lymphoprep (Axis-Shield, Norway). Pooled PBMC could be frozen in 10 % DMSO (Sigma) and stored at -80°C.

### Preparation of THP-1 cells

THP-1 cells were cultured in a humified incubator (5% CO2- 37°C) in complete RPMI (+glutamax, 10% fetal calf serum, 1% Penicillin-Streptomycin) (Invitrogen, CA, US). THP-1 cells were spun and washed once with PBS (22°C 300xg 7 min) and resuspended in PBS to 1x10⁷ cells/ml. The cells are stimulated with 0.1 µg PMA(Sigma)/10⁷ cells (10 µl of a 100xPBS diluted stock of 1 mg/ml) 15 min at RT. The cells are washed once in PBS.

### Combined ADCC and phagocytosis assay

This assay was adapted from Berkman et al. 2002. Autoimmunity 35, 415-419. Briefly, RhD positive (RhD+) or RhD negative (RhD-) red blood cells (RBC) were labeled with radioactive Chromium. For Cr51 labeling, 1 x 10⁸ RhD+ and RhD- RBC, respectively, were centrifuged (700xg for 2 min) and 100 µl RPMI ((Invitrogen, CA, US)) and 200 µl sodium chromate (0.2 µCi) (GE Healthcare, UK) were added to each tube before incubation for 1.5 hours at 37°C. The suspension was centrifuged (2 min 700xg) and supernatant removed. Then the RBCs were washed twice in 15 ml PBS and resuspended in PBS with 0.1% BSA (Sigma). Cells were adjusted to 2 x 10⁶ cells/ml and 50 µl/well were added to 96-well cell culture plates (Nunc). Fifty µl of two-fold dilutions in PBS with 0.1% BSA of Anti-RhD rpAb from Batch 1 (Sym001 rWS (research working standard) further described in WO 2006/007850 A1 Example 5), Batch 2, Batch 3, and Batch 4, was then added to each well, except control wells. The plates incubate 40 min at 37°C in the heating cupboard. Hereafter the cells are carefully washed (2 min 700xg) three times in 200 µl/well PBS and resuspended in 100 µl/well complete RPMI.

The PBMC were adjusted to 2 x 10⁷ cells/ml and 100 µl were added to each well. Whereas the THP-1 cells were adjusted to 1 x 10⁷ cells/ml and 100 µl were added to each well. Control wells were supplied with complete RPMI and used for either spontaneous lysis/retention or maximum lysis. The plate was incubated at 37°C overnight in a humified incubator.
One hundred µl 1% Triton-X-100 (Merck, Germany) was added to the maximum lysis control wells. The plates were centrifuged (700xg for 2 min) and 50 µl of the supernatant was transferred to ADCC Lumaplates (Perkin Elmer, Belgium).

Following transfer of the supernatants, the cell culture plates were centrifuged (700xg for 2 min) and 50 µl supernatant from the maximum lysis wells were transferred to another LumaPlate (phagocytosis LumaPlate). In the cell culture plate, the supernatant was removed from the remaining wells and lysis buffer (140 mM NH4Cl, 17 mM Tris-HCl) was added, followed by 5 min incubation at 37°C. NH4Cl lyses the RBC, but leaves the PBMC/THP-1 fraction and thereby the phagocytozed RBC intact. After RBC lysis, the plates were centrifuged (700xg for 2 min), pellets were washed twice in PBS, and resuspended in 100 µl PBS. One hundred µl 1% Triton-X-100 was added to the wells to lyse the phagocytic PBMC/THP-1, and 50 µl of the lysate was transferred to the phagocytosis LumaPlates. The LumaPlates were dried overnight at 37°C and counted in a TopCount NXT (Packard, CT, USA). All data were imported into Excel and analyzed as described by Berkman et al. 2002. Autoimmunity 35, 415-419. Briefly, the computations were performed as follows:
ADCC: Immune lysis (%) = (mean test Cr51 released - mean spontaneous Cr51 released) / (total Cr51 in target erythrocytes- machine background) x 100
Phagocytosis: Immune phagocytosis (%) = (mean test Cr51 retention - mean spontaneous Cr51 retention) / (total Cr51 in target erythrocytes- machine background) x 100
All data were normalized to the combined maximum plateau values

The functional activity of anti-RhD rpAb from the four consecutive reactor runs showed nearly identical functional activity in both in vitro assays with both effector cells (Fig. 2, 3, 4 and 5) reflecting the high consistency between the batches. However it is clear that the data generated by the THP-1 cell line contains less variability and a much higher dynamic range.

## Claims

1. A method for determining the potency of an antibody, said method comprising the steps of:
a) providing RhD(+) red blood cells comprising a detectable label,
b) providing at least one anti-RhD antibody the potency of which it is desirable to determine,
c) contacting said RhD(+) red blood cells with said at least one anti-RhD antibody, thereby obtaining a mixture of said RhD(+) red blood cells and said at least one anti-RhD antibody,
d) providing phagocytotic cells, said phagocytotic cells being non-adherent and said phagocytotic cells having been cultured in vitro,
e) contacting said mixture of said RhD(+) red blood cells and said at least one anti-RhD antibody with said phagocytotic cells, and
f) measuring the amount of label released from said RhD(+) red blood cells and/or the amount of label taken up by said phagocytotic cells,
wherein said amount of label released and/or said amount of label taken up is a measure of the potency of said at least one anti-RhD antibody.

2. A method for monitoring the release of label from a RhD(+) red blood cell and/or the amount of label taken up by phagocytotic cells, said phagocytotic cells being non-adherent and said phagocytotic cells having been cultured in vitro, said method comprising the steps of:
a) providing RhD(+) red blood cells comprising a detectable label,
b) providing at least one anti-RhD antibody,
c) contacting said RhD(+) red blood cells with said at least one anti-RhD antibody, thereby obtaining a mixture of said RhD(+) red blood cells and said at least one anti-RhD antibody,
d) providing phagocytotic cells, said phagocytotic cells being non-adherent and said phagocytotic cells having been cultured in vitro,
e) contacting said mixture of said RhD(+) red blood cells and said at least one anti-RhD antibody with said phagocytotic cells, and
f) measuring the amount of label released from said RhD(+) red blood cells and/or the amount of label taken up by said phagocytotic cells.

3. A method for determining the ability of an antibody to induce the release of label from a RhD(+) red blood cell comprising a detectable label and/or the ability to induce the uptake of label from a RhD(+) red blood cell comprising a detectable label a by phagocytotic cells, said phagocytotic cells having been cultured in vitro, said method comprising the steps of:
a) providing RhD(+) red blood cells comprising a detectable label,
b) providing at least one anti-RhD antibody,
c) contacting said RhD(+) red blood cells with said at least one anti-RhD antibody, thereby obtaining a mixture of said RhD(+) red blood cells and said at least one anti-RhD antibody,
d) providing phagocytotic cells, said phagocytotic cells being non-adherent and said phagocytotic cells having been cultured in vitro,
e) contacting said mixture of said RhD(+) red blood cells and said at least one anti-RhD antibody with said phagocytotic cells, and
f) measuring the amount of label released from said RhD(+) red blood cells and/or the amount of label taken up by said phagocytotic cells.

4. A method for determining whether a manufactured anti-D antibody fulfils a predefined release criterion, said method comprising the steps of:
a) providing RhD(+) red blood cells comprising a detectable label,
b) providing at least one anti-RhD antibody the potency of which it is desirable to determine,
c) contacting said RhD(+) red blood cells with said at least one anti-RhD antibody, thereby obtaining a mixture of said RhD(+) red blood cells and said at least one anti-RhD antibody,
d) providing phagocytotic cells, said phagocytotic cells being non-adherent and said phagocytotic cells having been cultured in vitro,
e) contacting said mixture of said RhD(+) red blood cells and said at least one anti-RhD antibody with said phagocytotic cells, and
f) measuring the amount of label released from said RhD(+) red blood cells and/or the amount of label taken up by said phagocytotic cells,
wherein said amount of label released and/or said amount of label taken up can be compared to said predetermined release criterion.

5. A method according to any of the preceding claims, wherein said RhD(+) red blood cells originate from a single donor.

6. A method according to any of the claims 1-4, wherein said RhD(+) red blood cells originate from a plurality of different donors.

7. A method according to any of the preceding claims, wherein said RhD(+) red blood cells have been frozen prior to use.

8. A method according to any of the claims 1-6, wherein said RhD(+) red blood cells have not been frozen prior to use.

9. A method according to any of the preceding claims, wherein said detectable label is chosen from the group consisting of: a radioactive label, a colour, a dye, a fluorescent label, a fluorochrome, a fluorophore, a heavy metal, haemoglobin, and an inherent label.

10. A method according to any of the preceding claims, wherein said detectable label is Chromium-51.

11. A method according to any of the preceding claims, wherein said at least one anti-RhD antibody is chosen from the group consisting of: monoclonal antibodies, polyclonal antibodies, recombinant antibodies, isolated immunoglobulins, binding fragments of antibodies, chimeric antibodies, full-length antibodies, and single chain antibodies.

12. A method according to any of the preceding claims, wherein said at least one anti-RhD antibody is a polyclonal recombinant antibody or a mixture of a plurality of different monoclonal antibodies.

13. A method according to any of the preceding claims, wherein said phagocytotic cells are a mixture of a plurality of different phagocytotic cells.

14. A method according to any of the preceding claims, wherein said phagocytotic cells are chosen from the group consisting of: monocytes, neutrophils, macrophages, granulocytes, and dendritic cells.

15. A method according to any of the preceding claims, wherein said phagocytotic cells are obtained from a cell line, preferably a monocytic cell line capable of effecting phagocytosis and/or ADCC.

16. A method according to claim 15, wherein said cell line is chosen from the group consisting of: ME-1, Mono Mac 6, NOMO-1, P31/Fujioka, P39/Tsugane, SCC-3, THP-1, and U-937, even more preferably from THP-1 or U937, yet more preferably THP-1.

17. A method according to any of the preceding claims, wherein said measuring of the amount of label released from said RhD(+) red blood cells and/or the amount of label taken up by said phagocytotic cells is carried out over at least 1 decade of antibody concentration, optionally at least 2 decades of antibody concentration, optionally at least 3 decades of antibody concentration, optionally at least 4 decades of antibody concentration, optionally at least 5 decades of antibody concentration.

18. A kit for measuring the potency of an antibody, said kit comprising:
a) a detectable label for labelling RhD(+) red blood cells,
b) phagocytotic cells, said phagocytotic cells being suitable for *in vitro* culture, and
c) a standard anti-RhD antibody composition with a pre-determined potency.

19. A kit according to claim 18, wherein said detectable label is chosen from the group consisting of: a radioactive label, a colour, a dye, a fluorescent label, a fluorochrome, a fluorophore, a heavy metal, haemoglobin, and an inherent label.

20. A kit according to claim 18 or 19, wherein said detectable label is Chromium-51.

21. A kit according to any of the claims 18-20, wherein said phagocytotic cells are a mixture of a plurality of different phagocytotic cells.

22. A kit according to any of the claims 18-21, wherein said phagocytotic cells are chosen from the group consisting of: monocytes, neutrophils, macrophages, granulocytes, and dendritic cells.

23. A kit according to any of the claims 18-22, wherein said phagocytotic cells are obtained from a cell line.

24. A kit according to claim 23, wherein said cell line is chosen from the group consisting of: monocytic cell line THP-1 and monocytic cell line U937.

25. A kit according to any of the claims 18-24, wherein said standard anti-RhD antibody composition is an International Reference Preparation.
